# EUROPEAN PATENT SPECIFICATION

(11) **EP 1 551 828 B1**
(45) Date of publication and mention of the grant of the patent: **23.01.2008**
(21) Application number: 03799022.3
(22) Date of filing: 24.09.2003
(51) Int. Cl.: C07D 403/06, C07D 471/04, A61K 31/415

(54) **PYRAZOLE DERIVATIVES**
PYRAZOLDERIVATE
DERIVES DE PYRAZOLE

(30) Priority: 07.10.2002 GB 0223232
(43) Date of publication of application: 13.07.2005
(73) Proprietor: Pfizer Limited, Sandwich, Kent CT13 9NJ (GB); PFIZER INC., New York, N.Y. 10017 (US)
(72) Inventor: Price, David Anthony, Sandwich, Kent CT13 9NJ (GB); Selby, Matthew Duncan, Sandwich, Kent CT13 9NJ (GB); Stupple, Paul Anthony, Sandwich, Kent CT13 9NJ (GB)
(74) Representative: Swarbrick, Terry Mark
(86) International application number: PCT/IB2003/004205
(87) International publication number: WO 2004/031178

(56) References cited:
- WO-A-02/04424
- WO-A-02/30907
- WO-A-02/085860
- GENIN M J ET AL: "NOVEL 1,5-DIPHENYLPYRAZOLE NONNUCLEOSIDE HIV-1 REVERSE TRANSCRIPTASE INHIBITORS WITH ENHANCED ACTIVITY VERSUS THE DELAVIRDINE-RESISTANT P236L MUTANT: LEAD IDENTIFICATION AND SAR OF 3- AND 4-SUBSTITUTED DERIVATIVES" JOURNAL OF MEDICINAL CHEMISTRY, AMERICAN CHEMICAL SOCIETY. WASHINGTON, US, vol. 43, 2000, pages 1034-1040, XP002178918 ISSN: 0022-2623

## Description

This invention relates to pyrazole derivatives, to their use in medicine, to compositions containing them, to processes for their preparation and to intermediates used in such processes.

Reverse transcriptase is implicated in the infectious lifecycle of Human Immunodeficiency Virus (HIV). Compounds which interfere with the function of this enzyme have shown utility in the treatment of conditions caused by HIV and genetically related retroviruses, such as Acquired Immune Deficiency Syndrome (AIDS). There is a constant need to provide new and better modulators, especially inhibitors, of HIV reverse transcriptase, since the virus is able to mutate, becoming resistant to the effects of known modulators.

Antiviral activity is ascribed to a class of N(hydroxyethyl)pyrazole derivatives in US patent number 3,303,200. A number of pyrazoles are disclosed as reverse transcriptase inhibitors, including: a class of N-phenylpyrazoles (J. Med. Chem., 2000, 43, 1034); a class of C and S linked aryl pyrazoles (WO02/04424); and a class of O and S linked aryl pyrazoles, the O and S aryl link being adjacent to the nitrogen atom (WO02/30907).

According to the present invention there is provided a compound of formula (I) 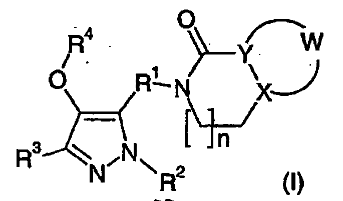 or a pharmaceutically acceptable salt or solvate thereof, wherein: W-X-Y defines a phenyl or pyridyl ring, said ring being optionally substituted by -CN;
R¹ is C₁-C₆ alkylene;
R² is H, C₁-C₆ alkyl, C₃-C₆ alkenyl, C₃-C₆ alkynyl, C₃-C₇ cycloalkyl, C₃-C₇ cycloalkenyl, phenyl, benzyl, R⁸ or R⁹, said C₁-C₆ alkyl, C₃-C₇ cycloalkyl, phenyl and benzyl being optionally substituted by halo, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, -NR⁵SO₂NR⁵R⁵, R⁸ or R⁹;
R³ is H, C₁-C₆ alkyl, C₃-C₇ cycloalkyl, phenyl, benzyl, halo, -CN, -OR⁷, -CO₂R⁵, -CONR⁵R⁵, R⁸ or R⁹, said C₁-C₆ alkyl, C₃-C₇ cycloalkyl, phenyl and benzyl being optionally substituted by halo, -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ or R⁹;
R⁴ is phenyl, naphthyl or pyridyl, each being optionally substituted by R⁸, halo, -CN, C₁-C₆ alkyl, C₁-C₆ haloalkyl, C₃-C₇ cycloalkyl, C₁-C₆ alkoxy, -CONR⁵R⁵, OR¹¹, SoₓR⁶, O-(C₁-C₆ alkylene)-CONR⁵R⁵, O-(C₁-C₆ alkylene)-NR⁵R⁵, or O-(C₁-C₆ alkylene)-OR⁶;
each R⁵ is independently either H, C₁-C₆ alkyl or C₃-C₇ cycloalkyl or, when two R⁵ groups are attached to the same nitrogen atom, those two groups taken together with the nitrogen atom to which they are attached represent azetidinyl, pyrrolidinyl, piperidinyl, homopiperidinyl, piperazinyl, homopiperazinyl or morpholinyl, said azetidinyl, pyrrolidinyl, piperidinyl, homopiperidinyl, piperazinyl, homopiperazinyl and morpholinyl being optionally substituted by C₁-C₆ alkyl or C₃-C₇ cycloalkyl;
each R⁶ is independently either H, C₁-C₆ alkyl or C₃-C₇ cycloalkyl;
R⁷ is C₁-C₆ alkyl or C₃-C₇ cycloalkyl;
R⁸ is a five or six-membered, aromatic heterocyclic group containing (i) from 1 to 4 nitrogen heteroatom(s) or (ii) 1 or 2 nitrogen heteroatom(s) and 1 oxygen or 1 sulphur heteroatom or (iii) 1 or 2 oxygen or sulphur heteroatom(s), said heterocyclic group being optionally substituted by halo, oxo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl;
R⁹ is a four to seven-membered, saturated or partially unsaturated heterocyclic group containing (i) 1 or 2 nitrogen heteroatom(s) or (ii) 1 nitrogen heteroatom and 1 oxygen or 1 sulphur heteroatom or (iii) 1 oxygen or sulphur heteroatom, said heterocyclic group being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN;
R¹⁰ is C₁-C₆ alkyl substituted by R⁸, R⁹, -OR⁵, -CONR⁵R⁵, -NR⁵COR⁵ or -NR⁵R⁵;
R¹¹ is phenyl optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl; and
x and n are independently 0, 1 or 2.

In the above definitions, halo means fluoro, chloro, bromo or iodo. Unless otherwise stated, alkyl, alkenyl, alkynyl, alkylene and alkoxy groups containing the requisite number of carbon atoms can be unbranched or branched chain. Examples of alkyl include methyl, ethyl, n-propyl, i-propyl, n-butyl, i-butyl, sec-butyl and t-butyl. Examples of alkenyl include ethenyl, propen-1-yl, propen-2-yl, propen-3-yl, 1-buten-1-yl, 1-buten-2-yl, 1-buten-3-yl, 1-buten-4-yl, 2-buten-1-yl, 2-buten-2-yl, 2-methylpropen-1-yl or 2-methylpropen-3-yl. Examples of alkynyl include ethynyl, propyn-1-yl,-propyn-3-yl, 1-butyn-1-yl, 1-butyn-3-yl, 1-butyn-4-yl, 2-butyn-1-yl. Examples of alkylene include methylene, 1,1-ethylene, 1,2-ethylene, 1,1-propylene, 1,2-propylene, 2,2-propylene and 1,3-propylene. Examples of alkoxy include methoxy, ethoxy, n-propoxy, i-propoxy, n-butoxy, i-butoxy, sec-butoxy and t-butoxy. Examples of cycloalkyl include, cyclopropyl, cyclobutyl, cyclopentyl, cyclohexyl and cycloheptyl. Where a heterocyclic group R⁸ or R⁹ is attached to an oxygen, sulphur or nitrogen heteroatom the heterocyclic group R⁸ or R⁹ must be linked through a ring carbon atom.

The pharmaceutically acceptable salts of the compounds of formula (I) include the acid addition and the base salts thereof.

Suitable acid addition salts are formed from acids which form non-toxic salts and examples are the hydrochloride, hydrobromide, hydroiodide, chloride, bromide, iodide, sulphate, bisulphate, nitrate, phosphate, hydrogen phosphate, acetate, fumarate, pamoate, aspartate, besylate, carbonate, bicarbonate/, camsylate, D and L-lactate, D and L-tartrate, esylate, mesylate, malonate, orotate, gluceptate, methylsulphate, stearate, glucuronate, 2-napsylate, tosylate, hibenzate, nicotinate, isethionate, matate, maleate, citrate, gluconate, succinate, saccharate, benzoate, esylate, and pamoate salts.

Suitable base salts are formed from bases which form non-toxic salts and examples are the sodium, potassium, aluminium, calcium, magnesium, zinc, choline, diolamine, olamine, arginine, glycine, tromethamine, benzathine, lysine, meglumine and diethylamine salts.

For reviews on suitable salts see Berge et al, J. Pharm. Sci., 66, 1-19, 1977 and Bighley et al, Encyclopedia of Pharmaceutical Technology, Marcel Dekker Inc, New York, 1996, Vol 13, pp453-497 The pharmaceutically acceptable solvates of the compounds of formula (I) include the hydrates thereof.

The compound of formula (I) may be modified to provide pharmaceutically acceptable derivatives thereof at any of the functional groups in the compound. Examples of such derivatives are described in: Drugs of Today, Volume 19, Number 9, 1983, pp 499 - 538; Topics in Chemistry, Chapter 31, pp 306 - 316; and in "Design of Prodrugs" by H. Bundgaard, Elsevier, 1985, Chapter 1 (the disclosures in which documents are incorporated herein by reference) and include: esters, carbonate esters, hemi-esters, phosphate esters, nitro esters, sulfate esters, sulfoxides, amides, sulphonamides, carbamates, azo-compounds, phosphamides, glycosides, ethers, acetals and ketals.

The invention encompasses all isomers of the compound of formula (I) and pharmaceutically acceptable salts or solvates thereof, including all geometric, tautomeric and optical forms, and mixtures thereof (e.g. racemic mixtures).

Separation of diastereoisomers may be achieved by conventional techniques, e.g. by fractional crystallisation, chromatography or high performance liquid chromatography (HPLC) of a stereoisomeric mixture of compounds. An individual enantiomer of a compound may also be prepared from a corresponding optically pure intermediate or by resolution, such as by HPLC of the corresponding racemate using a suitable chiral support, or by fractional crystallisation of the diastereoisomeric salts formed by reaction of the corresponding racemate with a suitable optically active acid or base, as appropriate.

The compound of formula (I) and pharmaceutically acceptable salts or solvates thereof may have the ability to crystallize in more than one form, a characteristic known as polymorphism, and all such polymorphic forms ("polymorphs") are encompassed within the scope of the invention. Polymorphism generally can occur as a response to changes in temperature or pressure or both, and can also result from variations in the crystallization process. Polymorphs can be distinguished by various physical characteristics, and typically the x-ray diffraction patterns, solubility behaviour, and melting point of the compound are used to distinguish polymorphs.

Compounds of formula (I), pharmaceutically acceptable salts and solvates thereof, isomers thereof, and polymorphs thereof, are hereinafter referred to as the compounds of the invention.

Preferred compounds of the invention are the compounds of formula (I) and pharmaceutically acceptable salts and solvates thereof.

Preferably, R¹ is methylene, ethylene or propylene.
Preferably, R¹ is methylene.

Preferably, R² is H, C₁-C₆ alkyl, C₃-C₆ alkenyl, phenyl, benzyl or R⁹, said phenyl, benzyl or C₁-C₆ alkyl being optionally substituted by halo, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹², -NR⁵COR⁵, -NR⁵COR⁸, -NR⁵COR¹², -NR⁵CO₂R⁵, -NR⁵CONR⁵R⁵, -SO₂NR⁵R⁵, -NR⁵SO₂R⁵, R⁸ or R⁹.
Preferably, R² is H, C₁-C₆ alkyl, phenyl or benzyl, said C₁-C₆ alkyl being optionally substituted by halo, -OR⁵, -OR¹⁰ or -CN.
Preferably, R² is H or C₁-C₃ alkyl.
Preferably, R² is H.

Preferably, R³ is H, C₁-C₆ alkyl or C₃-C₇ cycloalkyl, said C₁-C₆ alkyl being optionally substituted by halo, -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ or R⁹.
Preferably, R³ is H or C₁-C₆ alkyl.
Preferably, R³ is H or C₁-C₄ alkyl.
Preferably, R³ is methyl or ethyl.

Preferably, R⁴ is phenyl optionally substituted by R⁸, halo, -CN, C₁-C₆ alkyl, C₁-C₆ haloalkyl, C₃-C₇ cycloalkyl or C₁-C₆ alkoxy.
Preferably, R⁴ is phenyl substituted by R³, halo, -CN, C₁-C₆ alkyl, or C₁-C₆ alkoxy.
Preferably, R⁴ is phenyl substituted by halo or -CN.
Preferably, R⁴ is phenyl substituted by chloro or -CN.
Preferably, R⁴ is 3,5-dicyanophenyl or 3-chloro-5-cyanophenyl.

Preferably, R⁸ is pyrrolyl, imidazolyl, pyrazolyl, 1,2,3-triazolyl, 1,2,4-triazolyl, tetrazolyl, oxazolyl, isoxazolyl, thiazolyl, isothiazolyl, 1,2,4-oxadiazolyl, 1,3,4-oxadiazolyl, furanyl, thienyl, pyridinyl, pyridazinyl, pyrimidinyl or pyrazinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OR⁵, -NR⁵R⁵ or C₁-C₆ alkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OH, -NH₂ or methyl.

Preferably, R⁹ is azetidinyl, tetrahydropyrrolyl, piperidinyl, azepinyl, oxetanyl, tetrahydrofuranyl, tetrahydropyranyl, oxepinyl, morpholinyl, piperazinyl or diazepinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN. Preferably, R⁹ is azetidinyl, piperidinyl, tetrahydrofuranyl, piperazinyl or morpholinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN.
Preferably, R⁹ is azetidinyl, piperidinyl, tetrahydrofuranyl, piperazinyl or morpholinyl, each being optionally substituted by C₁-C₆ alkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by -OR⁵ or -NR⁵COR⁵. Preferably, R⁹ is azetidinyl, piperidinyl, tetrahydrofuranyl, piperazinyl or morpholinyl, each being optionally substituted by -CH₃, -SO₂CH₃, -CONH₂, -COOCH₃, -COCH₂OCH₃ or -COCH₃ and optionally substituted on a carbon atom which is not adjacent to a heteroatom by -OCH₃ or -NHCOCH₃.

Preferably, R¹⁰ is C₁-C₄ alkyl substituted by R⁸, R⁹, -OR⁵, -CONR⁵R⁵, -NR⁵COR⁵ or -NR⁵R⁵.
Preferably, R¹⁰ is C₁-C₄ alkyl substituted by R⁹, -OR⁵, -NR⁵COR⁵ or -NR⁵R⁵.
Preferably, R¹⁰ is C₁-C₂ alkyl substituted by tetrahydrofuranyl, -OCH₃, -NHCOCH₃ or -NH₂.

Preferably, R¹¹ is phenyl substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl.
Preferably, R¹¹ is phenyl substituted by halo, -CN, -CONR⁵R⁵, -SO₂NR-⁵R⁵ or -OR⁵.
Preferably, R¹¹ is phenyl substituted by fluoro, -CN, -CONH₂, -SO₂NH₂ or -OCH₃.

Preferably, n is 0 or 1.
Preferably, n is 0.

Preferred groups of compounds according to the invention include all combinations of the preferred definitions for individual substituents given above.

Preferred compounds of the invention are:
3-chloro-5-[3-methyl-5-(1-oxo-1,3-dihydro-isoindol-2ylmethyl)-1H-pyrazol-4-yloxy]-benzonitrile;
5-[3-methyl-5-(1-oxo-1,3-dihydro-isoindol-2-ylmethyl)-1H-pyrazol-4-yloxy]-isophthalonitrile;
and pharmaceutically acceptable salts, solvates or derivatives thereof.

The compounds of the invention may have advantages over those of the prior art with regard-to a number of useful properties or combinations thereof, such as potency, duration of action, pharmacokinetics, spectrum of activity, side effect profile, solubility, chemical stability, and so on.

The compounds of the invention may be prepared by any method known in the art for the preparation of compounds of analogous structure. The compounds of the invention can be prepared by the procedures described in the methods below, or by the specific methods described in the Examples, or by similar methods to either. The invention also encompasses any one or more of these processes for preparing the compounds of the invention, in addition to any novel intermediates used therein.

In the following methods, W, X, Y, R¹ to R⁴, and n are as previously defined for a compound of formula (I), unless otherwise stated; Z is H or C₁-C₄ alkoxy (e.g. methoxy); THF is tetrahydrofuran; DCM is dichloromethane; DMF is N,N-dimethylformamide and Ac is acyl.

Compounds of formula (I) may be prepared according to Scheme 1 that follows.

According to Scheme 1, compounds of formula (I) may be prepared by the reaction of a compound of formula (II) with an amine of formula (IV) under conventional conditions. For aldehydes of formula (II), i.e. wherein Z is H, reaction conditions are those of reductive amination/alkylation in the presence of a reducing agent. For esters of formula (II), i.e. wherein Z is C₁-C₄ alkoxy (e.g. methoxy), reaction conditions are those of alkylation/condensation in the presence of a base.

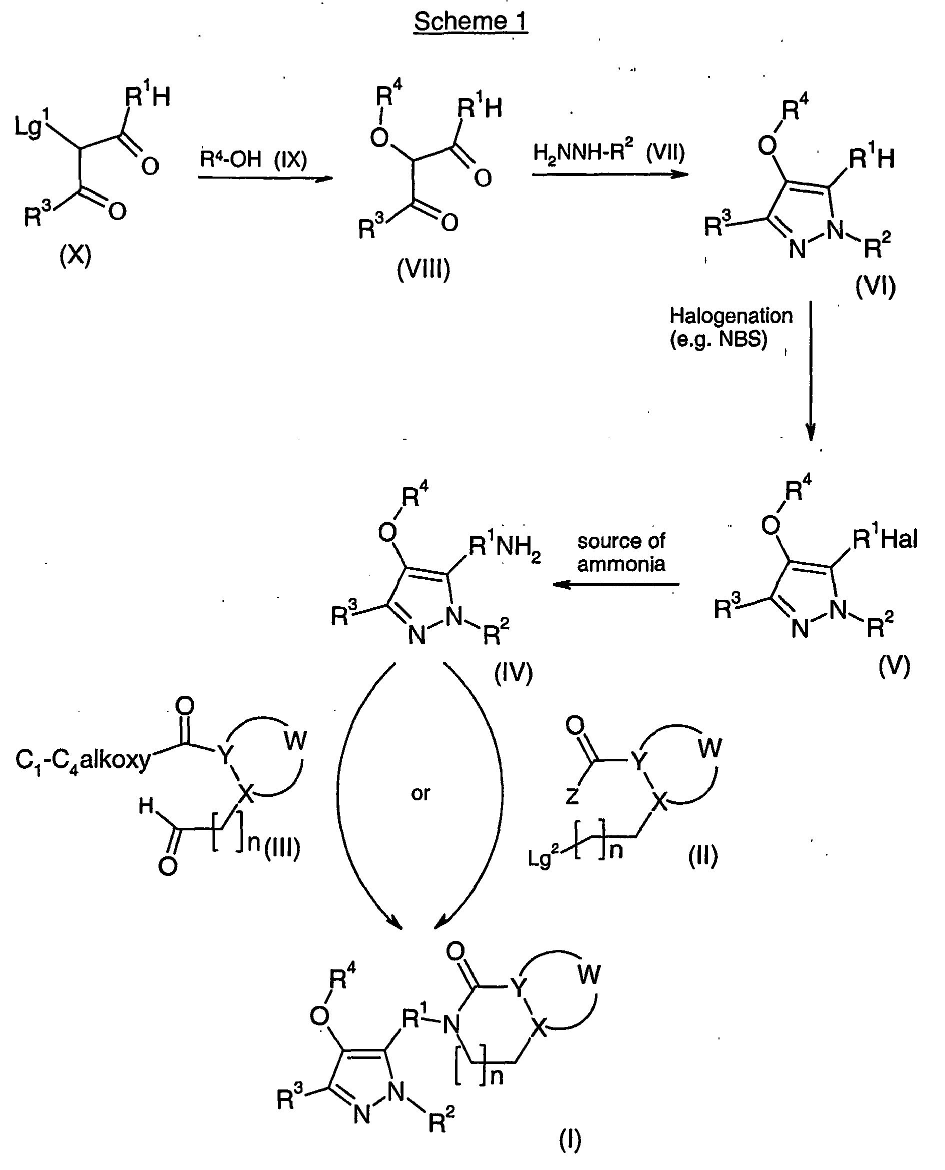

Conveniently, reductive amination/alkylation is effected using a hydride reducing agent, such as a borohydride (e.g. Na(OAc)₃BH or NaCNBH₃); optionally, an activating agent, such as acetic acid or sodium acetate; in the presence of a solvent, such as an ether (e.g. THF) or a haloalkane (e.g. DCM); and at ambient to elevated temperature, such as ambient temperature.

Conveniently, alkylation/condensation is effected using an alkali metal base, such as an alkali metal carbonate (e.g. sodium, potassium or caesium carbonate); in the presence of a solvent, such as a polar aprotic solvent (e.g. acetonitrile or DMF); and at ambient to elevated temperature, such as ambient temperature to 40°C.

Amines of formula (IV) may be prepared by reaction of the corresponding halide of formula (V) with a source of ammonia under conventional conditions. Conveniently the reaction is effected in the presence a solvent, such as an alcohol (e.g. ethanol or isopropanol), said solvent being saturated with ammonia; and at reduced to ambient to elevated temperature, such as reduced temperature (e.g. 0°C).

Compounds of formula (V) may be prepared by halogenation of a compound of formula (VI) using a source of halogen, such as a molecular halogen (e.g. bromine) or an N-halo-succinimide (e.g. N-bromo-succinimide), under conventional conditions. Conveniently the halogenation is effected in the presence of a solvent, such as a haloalkane (e.g. carbon tetrachloride or 1,1,1-trichloroethane); optionally a radical initiation catalyst, such as ultraviolet light or AIBN; and at ambient to elevated temperature, such as under reflux.

Compounds of formula (VI) may be prepared by the reaction of a compound of formula (VIII) with a hydrazine of formula (VII), or a salt or hydrate thereof. Conveniently, the reaction is effected a solvent, such as a protic solvent (e.g. acetic acid); at ambient to elevated temperature, such as ambient temperature; and optionally in the presence of an acid (e.g. acetic acid) or a base, such as a tertiary amine (e.g. triethylamine).

Compounds of formula (VIII) may be prepared by the reaction of a compound of formula (X) with an alcohol of formula (IX). Conveniently, the reaction is effected in the presence of a solvent, such as a polar solvent (e.g. acetone); a base, such as an inorganic base, preferably a metal carbonate (e.g. potassium or caesium carbonate); optionally, a nucleophilic catalyst, such as sodium iodide or tetrabutylammonium iodide; and at ambient to elevated temperature, such as elevated temperature (e.g. under reflux).

Ketoesters of formula (IX) are either commercially available, known in the literature, or may be prepared by conventional methods (e.g., where Lg¹ is Cl, by the chlorination of corresponding ketoesters, for instance using sulphonyl chloride).

Compounds of formula (I) may also be prepared by reaction of a compound of formula (III) with an amine of formula (IV) under conventional conditions. Conveniently, the reaction is effected in the presence of a reducing agent under conditions of reductive amination/alkylation, such as those described above for the preparation of a compound of formula (I) by reaction of a compound of formula (II) with an amine of formula (IV).

Compounds of formula (I) in which R³ is halo can be prepared from a compound of formula (XI) 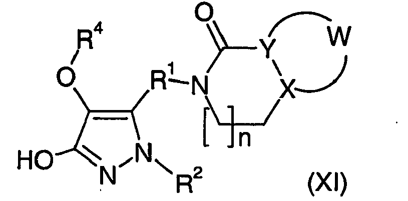 under conventional conditions. Conveniently, the reaction is effected by an inorganic acid halide, such as an inorganic acid chloride (e.g. POCl₃); optionally in the presence of a solvent, such as a polar aprotic solvent (e.g. N,N-dimethylformamide); and at reduced to ambient temperature, such as ambient temperature.

Compounds of formula (XI) may be prepared using the routes described above, *mutatis mutandis.*

It will be appreciated by those skilled in the art that, in many cases, compounds of formula (I) may be converted into other compounds of formula (I) by functional group transformations, including for example the following interconversions.

Compounds of formula (I) in which R² is optionally substituted C₁-C₆ alkyl may be prepared from compounds of formula (I) in R² is H by reaction with an alkylating agent. Suitable alkylating agents include bromoacetonitrile, ethyl 4-chloroacetoacetate, methyl bromoacetate and chloroethylamine hydrochloride. Conveniently, alkylation is effected in the presence of a suitable solvent, such as an alcohol (e.g. ethanol) or a polar aprotic solvent (e.g. N,N-dimethylformamide); a base, such as a metal hydride (e.g. sodium hydride) or metal alkoxide (e.g. sodium ethoxide); and at ambient to elevated temperature, such as under reflux.

Compounds of formula (I) in which R² or R³ contains a hydroxy group may be prepared from the corresponding compound of formula (I) in which R² or R³ contains an ester group by reduction. Conveniently, the reduction is effected by a metal hydride agent, such as lithium aluminium hydride; in a solvent, such as an ether (e.g. diethyl ether); and at reduced temperature, such as from -78°C to 0°C.

Compounds of formula (I) in which R² or R³ are substituted by a heterocycle of formula R⁸ and R⁹ may be prepared by standard heterocycle-forming reactions well known to the skilled man (see, for example, Advanced Organic Chemistry, 3rd Edition, by Gerry March or Comprehensive Heterocyclic Chemistry, A.R. Katritzky, C.W. Rees, E.F.V. Scriven, Volumes 1-11).

Compounds of formula (I) in which R³ is -CO₂H may be prepared by hydrolysis of a corresponding compound of formula (I) in which R³ is -CO₂R⁵. Conveniently, the reaction is effected in the presence of a solvent, such as an alcohol (e.g. aqueous ethanol), or an ether (e.g. aqueous 1,4-dioxan); and in the presence of a base, such as a metal hydroxide (e.g. sodium hydroxide). The skilled artisan will appreciate that such an acid may be converted into a primary amide by reaction with ammonia and a suitable coupling agent, such as a carbodiimide, e.g. dicyclohexylcarbodiimide, and that such a primary amide may then be converted into a nitrile by dehydration with a suitable dehydrating agent, such as phosphoryl chloride.

Compounds of formula (I) in which R³ is C₁-C₆ alkyl may be converted into the compounds of formula (I) in which R³ is C₁-C₆ alkyl substituted by halo (such as bromo), by halogenation, using a suitable halogenating agent. Conveniently the reaction is effected in the presence of a solvent, such as a haloalkane (e.g. dichloromethane) and at ambient temperature. Suitable halogenating agents include halogens (e.g. bromine) or N-halosuccinimides (e.g. N-bromsuccinimide).

Compounds of formula (I) containing an -OH, -NH- or -NH₂ group may be prepared by the deprotection of the corresponding compound bearing an -OP¹, -NP¹- or -NHP¹ group, respectively, wherein the group P¹ is a suitable protecting group. Examples of suitable protecting groups will be apparent to the skilled person; see, for instance, 'Protecting groups in Organic Synthesis (Second Edition)' by Theodora W. Green and Peter G. M. Wuts, 1991, John Wiley and Sons. Such compounds bearing an -OP¹, -NP¹- or -NHP¹ group may be prepared using the routes described above, *mutatis mutandis*.

Compounds of formulae (II), (III), (VII) and (IX) are either commercially available, known in the literature or easily prepared by methods well known to those skilled in the art, such as those described in the Preparations hereinafter.

The compounds of the invention can be administered alone, but will generally be administered in admixture with a suitable pharmaceutical excipient, diluent or carrier selected with regard to the intended route of administration and standard pharmaceutical practice.

For example, the compounds of the invention can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the invention may also be administered as fast-dispersing or fast-dissolving dosage forms or in the form of a high energy dispersion or as coated particles. Suitable formulations of the compounds of the invention may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

### General Example

A formulation of the tablet could typically contain from 0.01 mg to 500mg of active compound whilst tablet fill weights may range from 50mg to 1000mg. An example of a formulation for a 10mg tablet is illustrated below:

| Ingredient | %w/w |
|---|---|
| Compound of the invention | 10.000* |
| Lactose | 64.125 |
| Starch | 21.375 |
| Croscarmellose sodium | 3.000 |
| Magnesium Stearate | 1.500 |

| | |
|---|---|
| * Quantity adjusted in accordance with drug activity. | |

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The compounds of the invention can also be administered parenterally, for example, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of the compounds of the invention will usually be from 0.01 to 30 mg/kg, preferably from 0.01 to 5 mg/kg (in single or divided doses).

Thus tablets or capsules of the compound of the invention may contain from 1 to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that, in the treatment of certain conditions the compounds of the invention may be taken as a single dose as needed or desired.

The compounds of invention can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Alternatively, the compounds of the invention can be administered in the form of a suppository or pessary, or they may be applied topically in the form of a gel, hydrogel, lotion, solution, cream, ointment or dusting powder. The compounds of the invention may also be dermally or transdermally administered, for example, by the use of a skin patch. They may also be administered by the pulmonary or rectal routes.

They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO91/11172, WO94/02518 and WO98/55148.

It is to be appreciated that all references herein to treatment include curative, palliative and prophylactic treatment.

Oral administration is preferred.

Included within the scope of the invention are embodiments comprising the coadministration of a compound of the invention with one or more additional therapeutic agents, and compositions containing a compound of the invention along with one or more additional therapeutic agents. Such a combination therapy is especially useful for the prevention and/or treatment of infection by HIV and related retroviruses which may evolve rapidly into strains resistant to any monotherapy. Alternatively, additional therapeutic agents may be desirable to treat diseases and conditions which result from or accompany the disease being treated with the compound of the invention. For example, in the treatment of an HIV or related retroviral infection, it may be desirable to additionally treat opportunistic infections, neoplasms and other conditions which occur as a result of the immuno-compromised state of the patient being treated.

Preferred combinations of the invention include simultaneous or sequential treatment with a compound of the invention and one or,more:
(a) reverse transcriptase inhibitors such as abacavir, adefovir, didanosine, lamivudine, stavudine, zalcitabine and zidovudine;
(b) non-nucleoside reverse transcriptase inhibitors such as capavirine, delavirdine, efavirenz, and nevirapine;
(c) HIV protease inhibitors such as indinivir, nelfinavir, ritonavir, and saquinavir;
(d) CCR5 antagonists such as TAK-779 or UK-427,857;
(e) CXCR4 antagonists such as AMD-31 00;
(f) integrase inhibitors, such as L-870,810 or S-1360;
(g) inhibitors of viral fusion such as T-20;
(h) investigational drugs such as trizivir, KNI-272, amprenavir, GW-33908, FTC, PMPA, MKC-442, MSC-204, MSH-372, DMP450, PNU-140690, ABT-378, KNI-764, DPC-083, TMC-120 or TMC-125;
(i) antifungal agents, such as fluconazole, itraconazole or voriconazole; or
(j) antibacterial agents, such as azithromycin.

The activity of the compounds of the invention as reverse transcriptase inhibitors may be measured using the following assay.

### Inhibition of HIV-1 reverse transcriptase enzyme

Using purified recombinant HIV-1 reverse transcriptase (RT, EC, 2.7.7.49) obtained by expression in Escherichia Coli, a 96-well plate assay system is established for assaying a large number of samples using either the Poly(rA)-oligo(dT) Reverse Transcriptase [3H]-SPA enzyme assay system (Amersham NK9020) or the [3H]-flashplate enzyme assay system (NEN - SMP 103) and following the manufacturer's recommendations. The compounds are dissolved in 100% DMSO and diluted with the appropriate buffer to a 5% final DMSO concentration. The inhibitory activity is expressed in percent inhibition relative to DMSO control. The concentration at which compound inhibits reverse transcriptase by 50% is expressed as the IC₅₀ of the compound.

The compounds of Examples 1 and 4, when tested according to the above procedure, had IC₅₀ values of, respectively, 76 and 505 nanomolar.

Thus the invention provides:
(i) a compound of formula (I) or a pharmaceutically acceptable salt or solvate thereof;
(ii) a process for the preparation of a compound of formula (I) or a pharmaceutically acceptable salt or solvate thereof;
(iii) a pharmaceutical composition including a compound of formula (I) or a pharmaceutically acceptable salt or solvate thereof, together with a pharmaceutically acceptable excipient, diluent or carrier;
(iv) a compound of formula (I) or a pharmaceutically acceptable salt, solvate or composition thereof, for use as a medicament;
(v) a compound of formula (I) or a pharmaceutical acceptable salt, solvate or composition thereof, for use as a reverse transcriptase Inhibitor or modulator;
(vi) a compound of formula (I) or a pharmaceutically acceptable salt, solvate or composition thereof, for use in the treatment of an HIV or genetically-related retroviral infection, or a resulting acquired immune deficiency syndrome (AIDS);
(vii) a use of the compound of formula (I) or of a pharmaceutically acceptable salt, solvate or composition thereof, for the manufacture of a medicament having reverse transcriptase inhibitory or modulating activity;
(viii) the use of a compound of formula (1) or of a pharmaceutically acceptable salt, solvate or composition thereof, for the manufacture of a medicament for the treatment of an HIV or genetically-related retroviral infection, or a resulting acquired immune deficiency syndrome (AIDS);

The following Examples illustrate the preparation of the compounds of formula (I). The synthesis of certain intermediates used therein are described in the Preparations section that follows the Examples.

¹H Nuclear magnetic resonance (NMR) spectra were in all cases consistent with the proposed structures. Characteristic chemical shifts (δ) are given in parts-per-million downfield from tetramethylsilane using conventional abbreviations for designation of major peaks: e.g. s, singlet; d, doublet; t, triplet; q, quartet; m, multiplet; br, broad. The following abbreviations have been used: HRMS, high resolution mass spectrometry; hplc, high performance liquid chromatography; nOe, nuclear Overhauser effect; m.p., melting point; CDCl₃, deuterochloroform; D₆-DMSO, deuterodimethylsulphoxide; CD₃OD, deuteromethanol. Where thin layer chromatography (TLC) has been used it refers to silica gel TLC using silica gel 60 F₂₅₄ plates, R_{f} is the distance travelled by a compound divided by the distance travelled by the solvent front on a TLC plate.

### Example 1

### 3-Chloro-5-[3-methyl-5-(1-oxo-1,3-dihydro-isoindol-2ylmethyl)-1H-pyrazol-4-yloxy]-benzonitrile

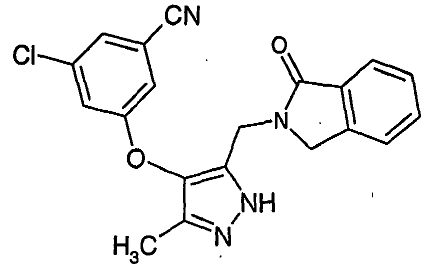

Sodium triacetoxyborohydride (93 mg, 0.44 mmol) was added to a solution of the amine of preparation 13 (107 mg, 0.4 mmol), methyl 2-formylbenzoate (Maybridge) (67 mg, 0.41 mmol) and acetic acid (23 µl, 0.4 mmol) in dichloromethane (2 ml), and the reaction stirred at room temperature for 1 hour. The mixture was diluted with dichloromethane (20 ml), and washed with sodium bicarbonate solution (10 ml). This aqueous solution was extracted with dichloromethane (2x10 ml), and the combined organic solutions were washed with brine (10 ml), dried over magnesium sulphate and evaporated under reduced pressure. The crude product was purified by chromatography on silica gel using an elution gradient of dichloromethane:methanol:0.88 ammonia (100:0:0 to 95:5:0.5) to afford the title compound as a white solid.
¹H NMR (400MHz, DMSO-d₆): δ 2.03 (s, 3H), 4.21 (s, 2H), 4.55 (s, 2H), 7.01 (d, 2H), 7.19 (s, 1 H), 7.40 (m, 4H).
LRMS: m/z ES+ 401 [M+Na]⁺.
Microanalysis found: C, 61.55; H, 4.07; N, 14.21. C₂₀H₁₅ClN₄O₂;0.5H₂O requires C, 61.94; H, 4.16; N, 14.45%.

### Example 2

### 3-Chloro-5-[3-methyl-5-(7-oxo-5,7-dihydro-pyrrolo[3,4-b]pyridin-6-ylmethyl)-1H-pyrazol-4-yloxy]-benzonitrile

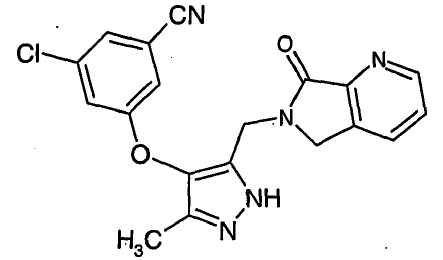

Sodium triacetoxyborohydride (65 mg, 0.3 mmol) followed by acetic acid (17 µl, 0.3 mmol) were added to the amine of preparation 13 (75 mg, 0.29 mmol) and the aldehyde from preparation 16 (60 mg, 3.1 mmol) in dichloromethane (5ml), and the reaction stirred at room temperature for 18 hours. The mixture was evaporated under reduced pressure and the residue purified by chromatography on silica gel using an elution gradient of dichloromethane:methanol:0.88 ammonia (100:0:0 to 90:10:1) to afford the title compound as a white solid (50 mg).
¹H NMR (400MHz, DMSO-d₆): δ 2.03 (s, 3H), 4.27 (s, 2H), 4.60 (s, 2H), 7.03 (s, 1H), 7.10 (s, 1H), 7.29 (s, 1H), 7.48 (m, 1H), 7.90 (m, 1H), 8.59 (m, 1H), 12.7 (br s, 1H).
LRMS: m/z APCI 380 [M+H]⁺.

### Example 3

### 3-Chloro-5-[3-methyl-5-(5-oxo-5,7-dihydro-pyrrolo[3,4-b]pyridin-6-ylmethyl)-1H-pyrazol-4-yloxy]-benzonitrile

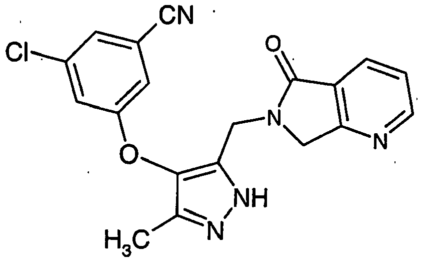

Sodium carbonate (100 mg, 0.91 mmol) followed by the bromide of preparation 15 (107 mg, 0.38 mmol) were added to a solution of the amine of preparation 13 (100 mg, 0.38 mmol) in N,N-dimethylformamide (5 ml), and the reaction stirred at room temperature for 3 hours. TLC analysis showed starting material remaining, so additional bromide (940 mg, 0.14 mmol) was added, and the reaction stirred for a further 4 days. The mixture was concentrated under reduced pressure, the residue azeotroped with toluene, then dissolved in ethyl acetate (50 ml). This solution was washed with water (2x30 ml) and brine (20 ml), then dried over magnesium sulphate and evaporated under reduced pressure.
The crude product was purified by chromatography on silica gel using an elution gradient of dichloromethane:methanol:0.88 ammonia (100:0:0 to 90:10:1). The product was then recrystallised from acetonitrile to afford the title compound (20 mg). mp-225-227°C
¹H NMR (400MHz, DMSO-d₆): δ 2.03 (s, 3H), 4.29 (s, 2H), 4.61 (s, 2H), 7.05 (s, 1 H), 7.11 (s, 1 H), 7.27 (s, 1 H), 7.40 (m, 1H), 7.82 (d, 1 H), 8.65 (d, 1 H), 12.69 (br s, 1H).
Microanalysis found: C, 59.00; H, 3.61; N, 18.19. C₁₉H₁₄ClN_{5O2};0.5H₂O requires C, 58.69; H, 3.89; N, 18.01 %.

### Example 4

### 5-[3-Methyl-5-(1-oxo-1,3-dihydro-isoindol-2-ylmethyl)-1H-pyrazol-4-yloxy]-isophthalonitrile

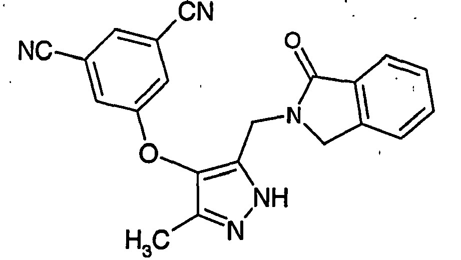

Sodium triacetoxyborohydride (110 mg, 0.52 mmol) was added to a solution of acetic acid (30 µl, 0.47 mmol), the amine from preparation 14 (120 mg, 0.47 mmol) and methyl 2-formylbenzoate (Maybridge) (81 mg, 0.5 mmol) in dichloromethane (5 ml) and tetrahydrofuran (3 ml) and the reaction stirred at room temperature for 2 hours. The reaction was diluted with dichloromethane (40 ml), and washed with sodium carbonate (10 ml) solution. This aqueous solution was extracted with further dichloromethane (20 ml), and the combined organic solutions then washed with brine, dried over magnesium sulphate and evaporated under reduced pressure. The residue was purified by chromatography on silica gel using an elution gradient of dichloromethane:methanol:0.88 ammonia (100:0:0 to 95:5:0.5) and the product recrystallised from ethyl acetate and acetonitrile to afford the title compound.
¹H NMR (400MHz, DMSO-d₆): δ 2.02 (s, 3H), 4.23 (s, 2H), 4.56 (s, 2H), 7.36-7.63 (m, 7H), 12.75 (br s, 1 H).
LRMS: m/z ES+ 392 [M+Na]⁺.
Mp-247-248°C
Microanalysis found: C, 67.69; H, 4.05; N, 18.77. C₂₁H₁₅N₅O₂;0.5H₂O requires C, 67.63; H, 4.16; N, 18.78%.

### Example 5

### 5-[5-(6-Cyano-1-oxo-1,3-dihydro-isoindol-2-ylmethyl)-3-methyl-1H-pyrazol-4-yloxy]-isophthalonitrile

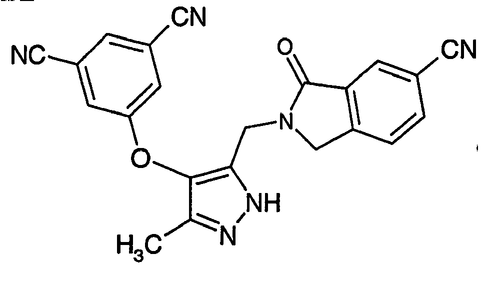

Sodium carbonate (301 mg, 2.84 mmol) was added to a suspension of the amine of preparation 14 (300 mg, 1.18 mmol) in N,N-dimethylformamide (10 ml), and the mixture stirred at 40°C for 30 minutes. 2-Bromomethyl-5-cyano-benzoic acid methyl ester (WO 0234745, line 12, pg 49) (300 mg, 1.18 mmol) was added, and the reaction stirred at 30°C for 18 hours. The mixture was concentrated under reduced pressure and the residue partitioned between ethyl acetate (25ml) and water (25ml). The resulting precipitate was filtered off and dried to afford the title compound as a white solid (160 mg).
¹H NMR (400MHz, DMSO-d₆): δ 2.00 (s, 3H), 4.40 (s, 2H), 4.60 (s, 2H), 7.50 (s, 2H), 7.60 (d, 1 H), 7.70 (s, 1 H), 7.80 (s, 1 H), 8.00 (d, 1H).
Microanalysis found: C, 65.38; H, 3.62; N, 20.64. C₂₂H₁₄N₆O₂;0.5H₂O requires C, 65.50; H, 3.75; N, 20.83%.

### Example 6

### 5-[5-(5-Cyano-1-oxo-1,3-dihydro-isoindol-2-ylmethyl)-3-methyl-1H-pyrazol-4-yloxy]-isophthalonitrile

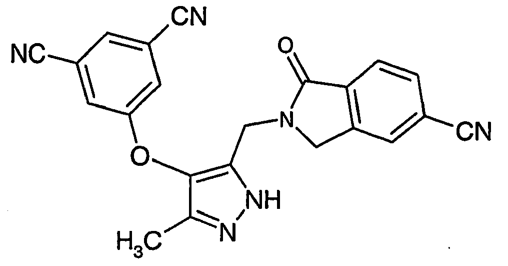

Sodium carbonate (401 mg, 3.78 mmol) was added to the amine from preparation 14 (400 mg, 1.6 mmol) in N,N-dimethylformamide (20 ml), followed by 2-bromomethyl-4-cyano-benzoic acid methyl ester (EP 685478, reference example 21) (340 mg, 1.6 mmol) and the reaction stirred at room temperature for 18 hours. The reaction was then warmed to 40°C for a further 3 hours, the mixture was concentrated under reduced pressure and the residue partitioned between ethyl acetate (25 ml) and water (25 ml). The layers were separated, the organic phase dried over magnesium sulphate and evaporated under reduced pressure. The crude product was purified by chromatography using a Bond Elut Si cartridge and dichloromethane:methanol:0.88 ammonia (100:0:0 to 90:10:1) as the eluant. The product was then triturated with hot acetonitrile, the solid filtered and dried to afford the title compound as a white solid (48.5mg).
¹H NMR (400MHz, DMSO-d₆): 2.02 (s, 3H), 4.36 (s, 2H), 4.60 (s, 2H), 7.42 (s, 2H), 7.50 (d, 1 H), 7.70 (s, 1 H), 7.82 (m, 1 H), 7.97 (s, 1 H), 12.75 (br s, 1H).
LRMS: m/z ES- 393 [M-H]⁻.
Microanalysis found: C, 65.55; H, 3.57; N, 21.03. C₂₂H₁₄N₆O₂;0.5H₂O requires C, 65.50; H, 3.75; N, 20.83%.

### Preparation 1

### 1-Bromo-3-chloro-5-methoxybenzene

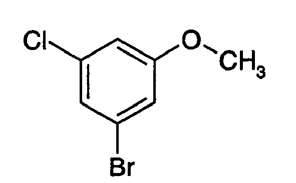

Sodium methoxide (4.5M solution in methanol, 2.20 ml, 10.0 mmol) was added dropwise to a stirred solution of 1-fluoro-3-chloro-5-bromobenzene (1.00 g, 4.77 mmol) in methanol (28 ml) at room temperature under a nitrogen atmosphere. The mixture was heated under reflux for 3 days and then cooled to room temperature. The mixture was evaporated under reduced pressure and the resulting yellow oil was dissolved in dichloromethane (30 ml). The dichloromethane solution was washed with water (2x20 ml) dried over magnesium sulphate, filtered and evaporated under reduced pressure. The residue was purified by chromatography on silica gel eluting with cyclohexane to provide the title compound as a colourless oil (302 mg).
¹H-NMR (400MHz, CDCl₃): δ 3.77 (s, 3H), 6.82 (s, 1 H), 6.94 (s, 1 H), 7.09 (s, 1H). Microanalysis: Found: C, 37.94; H, 2.75. C₇H₆BrClO requires; C, 37.96; H, 2.73%.

### Preparation 2

### 1,3-Dibromo-5-methoxybenzene

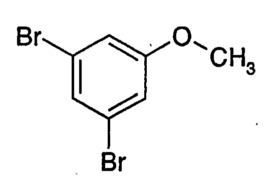

Sodium methoxide (4.5M solution in methanol, 8.80 ml, 41.0 mmol) was added dropwise to a stirred solution of 3,5-dibromofluorobenzene (5.00 g, 19.0 mmol) in *N,N*-dimethylformamide (95 ml) at 0°C under a nitrogen atmosphere. The reaction was warmed to room temperature, stirred for 1 hour and then evaporated under reduced pressure. The residue was dissolved in diethyl ether and was washed with water (3x300 ml) and brine (300 ml), dried over magnesium sulphate, filtered and concentrated under reduced pressure to give the title compound as a white solid (5.13 g).
¹H-NMR (300MHz, CDCl₃): δ 3.79 (s, 3H), 7.00 (s, 2H), 7.26 (s, 1H).
LRMS: m/z TS+ 266 [M+H]⁺.

### Preparation 3

### 3-Chloro-5-methoxybenzonitrile

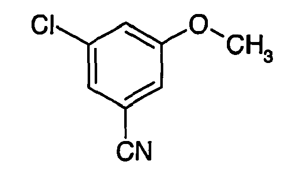

Tetrakis(triphenylphosphine)palladium (0) (174 mg, 0.15 mmol) was added in one portion to a stirred solution of the bromide of Preparation 1 (500 mg, 2.26 mmol) and zinc cyanide (146 mg, 1.24 mmol) in *N*,*N*-dimethylformamide (3 ml) at room temperature under a nitrogen atmosphere. The reaction was heated at 100°C for 14 hours and then cooled to room temperature. The solvent was evaporated under reduced pressure and the residue was purified by chromatography on silica gel using ethyl acetate in cyclohexane as eluant (5:95) to provide the title compound as a yellow oil (380 mg).
¹H-NMR (300MHz, CDCl₃): δ 3.82 (s, 3H), 7.04 (s, 1 H), 7.12 (s, 1 H), 7.23 (s, 1H).

### Preparation 4

### 3,5-Dicyanomethoxybenzene

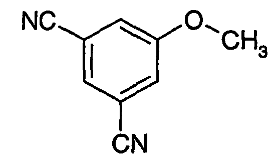

Tris(dibenzylideneacetone)dipalladium(O) (6.53g, 7.15mmol) was added in one portion to a stirred solution of the bromide of Preparation 2 (38.0g, 143mmol), 1,1'-bis(diphenylphosphino)ferrocene (9.3g, 16.8mmol) and zinc cyanide (20.0g, 172mmol) in *N*,*N*-dimethylformamide (300ml) at room temperature under nitrogen. The reaction was heated at 100°C for 14 hours and cooled to room temperature. Water (1500ml) was added and the mixture was extracted with ethyl acetate (3x500ml). The combined organics were filtered and the filtrate was washed with water (500ml), dried over magnesium sulphate, filtered and concentrated under reduced pressure. The resulting solid was triturated with toluene (1000ml) to provide the title compound (18.0g) as a tan solid.
¹H-NMR (300MHz, CDCl₃): δ 3.83 (s, 3H), 7.31 (s, 2H), 7.48 (s, 1H).

### Preparation 5

### 3-Chloro-5-hydroxybenzonitrile

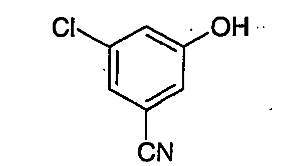

Boron trichloride (1.OM solution in dichloromethane, 26.0 ml, 26.0 mmol) was added dropwise to a stirred solution of the nitrile of Preparation 3 (1.80 g, 10.0 mmol) and tetrabutylammonium iodide (4.36 g, 11.0 mmol) in dichloromethane (50 ml) at -78°C. The reaction mixture was warmed to room temperature and stirred for 14 hours. The reaction mixture was cooled to 0°C and ice and dichloromethane (100 ml) were added. The organic phase was washed with water (3x40 ml) and brine (40 ml), dried over magnesium sulphate, filtered and concentrated under reduced, pressure. The residue was purified by chromatography on silica gel using ethyl acetate in cyclohexane as eluant (20:80) to give the title compound as a white solid (900 mg).
¹H-NMR (400MHz, DMSO-d₆): δ 7.12 (m, 2H), 7.38 (s, 1 H), 10.65 (s, 1H).
Microanalysis: Found: C, 54.76; H, 2.81; N, 8.94. C₇H₄CINO requires; C, 54.75; H, 2.63; N, 9.12%.

### Preparation 6

### 3,5-Dicyanohydroxybenzene

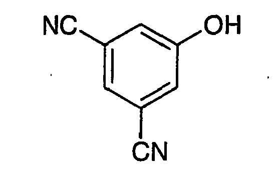

The ether of Preparation 4 (9.60 g, 60.7 mmol) was added portionwise to a stirred suspension of aluminium trichloride (32.4 g, 243 mmol) in dichloromethane (250 ml) at 0°C under a nitrogen atmosphere. The suspension was stirred at 45°C for 6 days, then cooled to room temperature and poured onto ice (450 ml). Concentrated hydrochloric acid (450 ml) was added dropwise and the resulting suspension was stirred for 10 minutes at room temperature. The solid formed was isolated by filtration, washed with water and dried over phosphorus pentoxide to give the title compound as a tan solid (7.83 g).
¹H-NMR (400MHz, CDCl₃): δ 7.36 (m, 2H), 7.56 (m, 1H).

### Preparation 7

### 3-[(3,5-Dimethyl-1H-pyrazol-4-yl)oxy]-5-chlorobenzonitrile

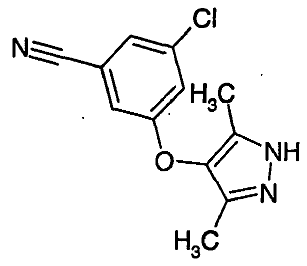

A mixture of 3-chloro-2,4-pentanedione (6.73 g, 50.0 mmol), the phenol of Preparation 5 (7.67 g, 50.0 mmol), caesium carbonate (18.0 g, 55.4 mmol) and acetone (40 ml) was heated under reflux for 2 hours. The reaction was cooled to room temperature, *N,N*-dimethylformamide (6 ml) and acetone (30 ml) were added and the reaction was heated at 70°C for a further 12 hours. The mixture was cooled to room temperature and the solid was removed by filtration and dissolved in 1 M aqueous hydrochloric acid (150 ml). The aqueous solution was extracted with dichloromethane (3x100 ml) and the combined organic phases were washed with brine (30 ml), dried over magnesium sulphate, filtered and concentrated under reduced pressure to give a brown solid (5.5 g).
This was suspended in acetic acid (22 ml), hydrazine hydrate (1.1 ml, 22 mmol) added, and the reaction stirred at room temperature for 18 hours. The mixture was concentrated under reduced pressure and the residue purified by chromatography on silica gel using an elution gradient of dichloromethane:ethyl acetate (100:0 to 85:15) to afford the title compound, (4.8 g).
¹H-NMR (400MHz, CDCl₃): δ 2.09 (s, 6H), 7.02 (m, 1 H), 7.10 (m, 1H), 7.25 (m, 1H).
Microanalysis found: C, 57.86; H, 4.03; N, 16.78. C₁₂H₁₀ClN₃O requires C, 58.19; H, 4.07; N, 16.97%.

### Preparation 8

### 5-(3,5-Dimethyl-1H-pyrazol-4-yloxy)-isophthalonitrile

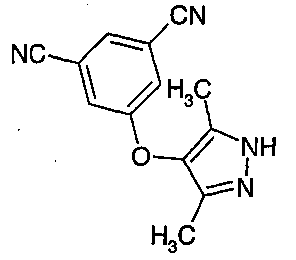

The phenol from preparation 6 (2 g, 13.8 mmol) was mixed with 3-chloro-2,4-pentanedione (2 ml, 16.7 mmol) and caesium carbonate (4.51 g, 13.8 mmol) in acetone (50ml), and was heated at 65°C for 2 hours. The mixture was cooled to room temperature and concentrated hydrochloric acid (2 ml) was added. The mixture was diluted with water (50 ml) and extracted with ethyl acetate (3x50 ml). The combined organic solutions were washed with brine, dried over magnesium sulphate and evaporated under reduced pressure. The residual yellow oil was dissolved in acetic acid (30 ml) and hydrazine (1 ml, 20.7 mmol) was added. The mixture was stirred at room temperature for 10 minutes and then evaporated under reduced pressure. The residue was dissolved in ethyl acetate (50 ml) and washed with 10% sodium carbonate solution (30 ml), water (30 ml), brine (30 ml) and then dried over magnesium sulphate. The solvent was evaporated under reduced pressure and the residue was triturated with diethyl ether to give the title compound as a pale yellow solid (1.8 g).
m.p.182-185°C; LRMS: m/z TS+ 239 [M+H]⁺
¹H-NMR (300MHz, CDCl₃): δ 2.16 (s, 6H), 7.40 (s, 2H), 7.59 (s, 1H).

### Preparation 9

### 3-[(1-Acetyl-3,5-dimethyl-1H-pyrazol-4-yl)oxy]-5-chlorobenzonitrile

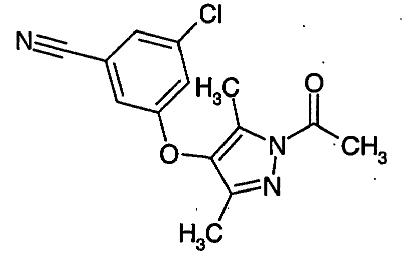

Sodium hydride (60% dispersion in oil, 840 mg, 21.0 mmol) was added to a stirred solution of acetyl chloride (1.50 ml, 21.0 mmol) and the pyrazole of Preparation 7 (4.80 g, 19.4 mmol) in *N*,*N*-dimethylformamide (20 ml) at 0°C under a nitrogen atmosphere. The reaction was stirred at 0°C for 15 minutes and then water (200 ml) was added. The aqueous mixture was extracted with ethyl acetate (3x120 ml). The combined organic phases were washed with water (50 ml) and brine (50 ml), dried over magnesium sulphate, filtered and evaporated under reduced pressure to leave a yellow solid. The crude product was purified by chromatography on silica gel using dichloromethane as eluant to give the title compound as a white solid (5.00 g).
¹H-NMR (400MHz, CDCl₃): δ 2.06 (s, 3H), 2.38 (s, 3H), 2.65 (s, 3H), 6.99 (m, 1 H), 7.08 (m, 1H), 7.29 (m, 1 H).
LRMS: m/z TS+ 290 [M+H]⁺.

### Preparation 10

### 5-[1-Acetyl-3,5-dimethyl-1H-pyrazol-4-yloxy]-isophthalonitrile

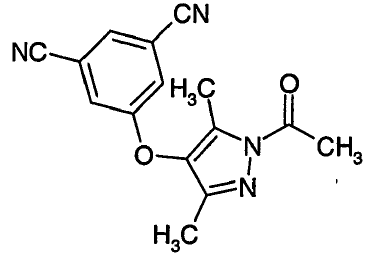

The pyrazole from preparation 8 (1.75 g, 7.35 mmol) was dissolved in N,N-dimethylformamide (15 ml) and was cooled to 0°C. Acetyl chloride (0.78 ml, 11.03 mmol) and then sodium hydride (60% in mineral oil, 441 mg, 11.03 mmol) were added. The mixture was stirred at 0°C for 15 minutes and then a saturated solution of ammonium chloride (10 ml) was added. The reaction mixture was extracted with ethyl acetate (3x50 ml) and the combined organic layers were washed with water (50 ml) and brine (200 ml) dried over magnesium sulphate and evaporated under reduced pressure. The residue was triturated with ether to afford the title compound as a white solid, (1.5g).
¹H-NMR (400MHz, CDCl₃): δ 2.06 (s, 3H), 2.38 (s, 3H), 2.66 (s, 3H), 7.33 (s, 2H), 7.56 (s, 1 H).
LRMS: m/z TS+ 281.2 [M+H]⁺.

### Preparation 11

### 3-{[1-Acetyl-3-(bromomethyl)-5-ethyl-1H-pyrazol-4-yl]oxy}-5-chlorobenzonitrile

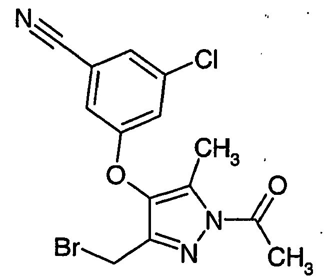

*N*-Bromosuccinimide (4.60 g, 25.6 mmol) was added to a stirred solution of the pyrazole of Preparation 9 (5.00 g, 17.3 mmol) and azobisisobutyronitrile (20 mg) in 1,1,1-trichloroethane (70 ml) at room temperature under a nitrogen atmosphere. The reaction was heated at 80°C for 3 hours and then cooled to room temperature. A second portion of *N*-bromosuccinimide (2.0 g, 11.2 mmol) was added and the reaction mixture was heated at 80°C for 4 hours. The reaction was cooled to room temperature, evaporated under reduced pressure and the resulting yellow oil was purified by chromatography on silica gel using pentane in dichloromethane as eluant (25:75) to give the title compound as a white solid (2.30 g).
m.p. 122-123°C.
¹H-NMR (400MHz, CDCl₃): δ 2.10 (s, 3H), 2.74 (s, 3H), 4.73 (s, 2H), 7.12 (s, 1H), 7.22 (s, 1H), 7.39 (s, 1H).

### Preparation 12

### 5-[1-Acetyl-5-bromomethyl-3-methyl-1H-pyrazol-4-yloxy]-isophthalonitrile

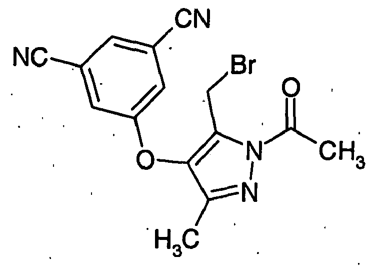

A solution of the pyrazole of preparation 10 (1.45 g, 5.18 mmol) and N-bromo succinimide (1.38 g, 7.76 mmol) in carbon tetrachloride (50 ml) was purged with nitrogen for 20 minutes. 2,2-Azobis(isobutyronitrile) (catalytic) was added and the mixture was heated at 95°C for 1 hour. The solvent was evaporated under reduced pressure and the residue was purified by chromatography on silica gel using ethyl acetate in pentane (80:20) as eluant to give the title compound as a white solid (1.7 g).
¹H-NMR (400MHz, CDCl₃): δ 2.06 (s, 3H), 2.66 (s, 3H), 4.67 (s, 2H), 7.40 (s, 2H), 7.63 (s, 1H).

### Preparation 13

### 3-(5-Aminomethyl-3-methyl-1H-pyrazol-4-yloxy)-5-chlorobenzonitrile

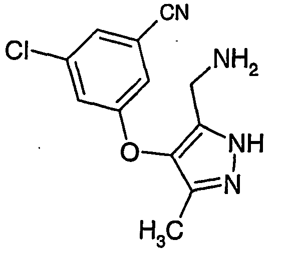

The bromide of Preparation 11 (300 mg, 0.80 mmol) was added to a saturated solution of ammonia in *iso*-propanol (50 ml) at 0°C. The reaction was stirred for 2 hours and allowed to slowly warm to room temperature. The mixture was concentrated under reduced pressure and the resulting yellow oil was dissolved in dichloromethane (50 ml). The dichloromethane was washed with 1 M aqueous sodium carbonate solution (20 ml), dried over magnesium sulphate, filtered and concentrated under reduced pressure to provide the title compound (220 mg) as a white foam.
¹H-NMR (300MHz, CDCl₃): δ 2.14 (s, 3H), 3.79 (s, 2H), 7.08 (s, 1 H), 7.16 (s, 1 H), 7.31 (s, 1 H).
LRMS (thermospray): m/z 263 [M+H⁺].

### Preparation 14

### 5-(5-Aminomethyl-3-methyl-1H-pyrazol-4-yloxy)-isophthalonitrile

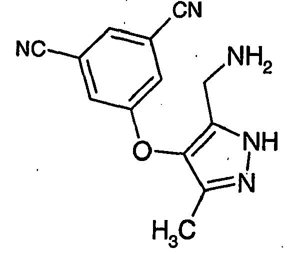

The bromide of preparation 12 (1 g, 2.8 mmol) was added to a freshly prepared solution of *iso*-propanol (100 ml) saturated with ammonia, and the reaction stirred at room temperature for 3 hours. The mixture was concentrated under reduced pressure and the residue partitioned between 10% aqueous potassium carbonate solution and dichloromethane. The aqueous phase was extracted further with dichloromethane and then ethyl acetate. The combined organic solutions were dried over magnesium sulphate and concentrated under reduced pressure. The crude product was purified by chromatography on silica gel using dichloromethane:methanol:0.88 ammonia (93:7:1) as eluant to afford the title compound as a white solid (316 mg).
¹H-NMR (400MHz, DMSO-d₆): δ 1.98 (s, 3H), 3.50 (s, 2H), 7.72 (s, 2H), 8.08 (s, 1H).
LRMS: m/z ES+ 254 [M+H]⁺.

### Preparation 15

### 2-Bromomethyl-pyridine-3-carboxylic acid ethyl ester hydrochloride

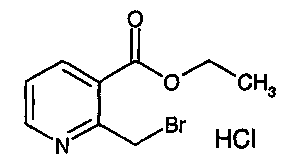

A mixture of ethyl 2-methylnicotinate (5.0 g, 30.3 mmol), N-bromosuccinimide (7.5 g, 42.1 mmol) and benzoyl peroxide (0.5 g) in carbon tetrachloride (100ml) was heated under reflux for 6 hours, then allowed to cool. The resulting mixture was filtered, the filtered solid washed with carbon tetrachloride, and the combined filtate washed with 4% sodium hydroxide solution, water and 2% hydrochloric acid, then dried over sodium sulphate. The solution was then treated with ethereal hydrochloric acid, and the resulting precipitate was filtered off, washed with ether and dried to afford the title compound as a pale yellow solid (4.5 g).
¹H NMR (60MHz, DMSO-d₆): δ 1.40 (t, 3H), 4.42 (q, 2H), 5.02-5.22 (m, 2H), 7.70 (m, 1H), 8.40 (m, 1 H), 8.80 (m, 1 H), 10.20 (s, 1H).

### Preparation 16

### 3-Formyl-pyridine-2-carboxylic acid ethyl ester

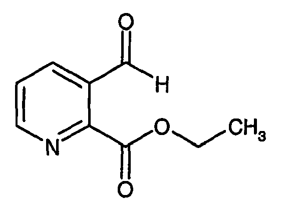

Triethylamine (1.4 ml, 10 mmol) was added to a solution of 2-bromopyridine-3-carbaldehyde (Synthesis 1999; (2); 306) (1.5 g, 8.0 mmol), tetrakis(triphenylphosphine)palladium (0) (470 mg, 0.4 mmol) in ethanol (50 ml), and this mixture heated to 100°C under an atmosphere of carbon monoxide in a sealed vessel for 16 hours. The cooled reaction mixture was concentrated under reduced pressure and the residue partitioned between ethyl acetate (50 ml) and 2N hydrochloric acid (20 ml). The layers were separated, the aqueous phase extracted with further ethyl acetate (50 ml), and the combined organic solutions dried over magnesium sulphate and evaporated under reduced pressure. The crude product was purified by chromatography on silica gel using an elution gradient of pentane:ethyl acetate (66:34 to 50:50) to afford the title compound, (130 mg).
¹H NMR (400MHz, CDCl₃): δ 1.48 (t, 3H), 4.53 (q, 2H), 7.61 (m, 1 H), 8.25 (d, 1 H), 8.87 (d, 1H), 10.60 (s, 1 H).

## Claims

1. A compound of formula (I) 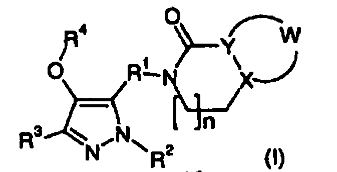 or a pharmaceutically acceptable salt or solvate thereof, wherein:
W-X-Y defines a phenyl or pyridyl ring, said ring being optionally substituted by -CN; R¹ is C₁-C₆ alkylene;
R² is H, C₁-C₆ alkyl, C₃-C₆ alkenyl, C₃-C₆ alkynyl, C₃-C₇ cycloalkyl, C₃-C₇ cycloalkenyl, phenyl, benzyl, R⁸ or R⁹, said C₁-C₆ alkyl, C₃-C₇ cycloalkyl, phenyl and benzyl being optionally substituted by halo, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵,-NR⁵SO₂R⁵,-NR⁵SO₂NR⁵R⁵, R⁸ or R⁹;
R³ is H, C₁-C₆ alkyl, C₃-C₇ cycloalkyl, phenyl, benzyl, halo, -CN, -OR⁷, -CO₂R⁵, -CONR⁵R⁵, R⁸ or R⁹, said C₁-C₆ alkyl, C₃-C₇ cycloalkyl, phenyl and benzyl being optionally substituted by halo, -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO²R⁵, R⁸ or R⁹;
R⁴ is phenyl, naphthyl or pyridyl, each being optionally substituted by R⁸, halo,
-CN, C₁-C₆ alkyl, C₁-C₆ haloalkyl, C₃-C₇ cycloalkyl, C₁-C₆ alkoxy, -CONR⁵R⁵, OR¹¹, SoₓR⁶, O-(C₁-C₆ alkylene)-CONR⁵R⁵, O-(C₁-C₆ alkylene)-NR⁵R⁵, or O(C₁-C₆ alkylene)-OR⁶;
each R⁵ is independently either H, C₁-C₆ alkyl or C₃-C₇ cycloalkyl or, when two R⁵ groups are attached to the same nitrogen atom, those two groups taken together with the nitrogen atom to which they are attached represent azetidinyl, pyrrolidinyl, piperidinyl, homopiperidinyl, piperazinyl, homopiperazinyl or morpholinyl, said azetidinyl, pyrrolidinyl, piperidinyl, homopiperidinyl, piperazinyl, homopiperazinyl and morpholinyl being optionally substituted by C₁-C₆ alkyl or C₃-C₇ cycloalkyl;
each R⁶ is Independently either H, C₁-C₆ alkyl or C₃-C₇ cycloalkyl;
R⁷ is C₁-C₆ alkyl or C₃-C₇ cycloalkyl;
R⁸ is a five or six-membered, aromatic heterocyclic group containing (i) from 1 to 4 nitrogen heteroatom(s) or (ii) 1 or 2 nitrogen heteroatom(s) and 1 oxygen or 1 sulphur heteroatom or (III) 1 or 2 oxygen or sulphur heteroatom(s), said heterocyclic group being optionally substituted by halo, oxo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl:
R⁹ is a four to seven-membered, saturated or partially unsaturated heterocyclic group containing (I) 1 or 2 nitrogen heteroatom(s) or (II) 1 nitrogen heteroatom and 1 oxygen or 1 sulphur heteroatom or (III) 1 oxygen or sulphur heteroatom, said heterocyclic group being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN;
R¹⁰ is C₁-C₆ alkyl substituted by R⁸, R⁹, -OR⁵, -CONR⁵R⁵, -NR⁵COR⁵ or -NR⁵R⁵; R¹¹ is phenyl optionally substituted by halo, -CN, -COR⁵, -CONR⁵R⁵, - SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl; and
x and n are independently 0,1 or 2.

2. A pharmaceutical composition Including a compound of formula (I) or a pharmaceutically acceptable salt or solvate thereof together with one or more pharmaceutically acceptable excipients, diluents or carriers.

3. A pharmaceutical composition according to claim 2 including one or more additional therapeutic agents.

4. A compound of formula (I) or a pharmaceutically acceptable salt or solvate thereof, or a pharmaceutical composition according to claim 2 or 3, for use as a medicament.

5. A compound of formula (I) or a pharmaceutically acceptable salt or solvate thereof, or a pharmaceutical composition according to claim 2 or 3, for use as a reverse transcriptase inhibitor or modulator.

6. A compound of formula (I) or a pharmaceutically acceptable salt or solvate thereof, or a pharmaceutical composition according to claim 2 or 3, for use in the treatment of an HIV or genetically-related retroviral infection, or a resulting acquired immune deficiency syndrome (AIDS).

7. Use of a compound of formula (I) or a pharmaceutically acceptable salt or solvate thereof, or a pharmaceutical composition according to claim 2 or 3, for the manufacture of a medicament having reverse transcriptase inhibitory or modulating activity.

8. Use of a compound of formula (I) or a pharmaceutically acceptable salt or solvate thereof, or a pharmaceutical composition according to claim 2 or 3, for the manufacture of a medicament for the treatment of an HIV or genetically-related retroviral infection, or a resulting acquired Immune deficiency syndrome (AIDS).

9. A process for preparing the compound of formula (I) or a pharmaceutically acceptable salt or solvate thereof, which comprises:
(A) reaction of a compound of formula (II) 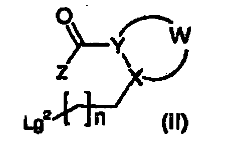 with an amine of formula (IV) 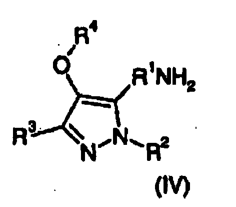 under conventional conditions;
(B) reaction of a compound of formula (III) 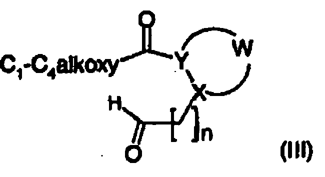 with an amine of formula (IV) 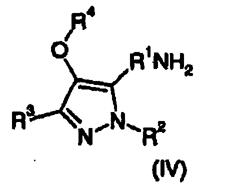 under conventional conditions;
(C) for the preparation of a compound of formula (I) in which R³ is halo, halogenating a compound of formula (XI) 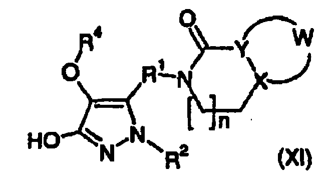 under conventional conditions;
(D) Interconversion of a compound of formula (I) Into another compound of formula (I); or
(E) deprotecting a protected derivative of compound of formula (I); and
optionally converting a compound of formula (I) prepared by any one of processes (A) to (E) Into pharmaceutically acceptable salt or solvate thereof.

## Patentansprüche

1. Verbindung der Formel (I) 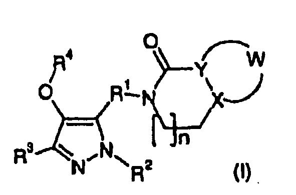 oder ein pharmazeutisch verträgliches Salz oder Solvat davon, worin:
W-X-Y einen Phenyl- oder Pyridylring definiert, wobei der Ring gegebenenfalls mit -CN substituiert ist;
R¹ C₁-C₆-Alkylen ist;
R² H, C₁-C₆-Alkyl, C₃-C₆-Alkenyl, C₃-C₆-Alkinyl, C₃-C₇-Cycloalkyl, C₃-C₇-Cycloalkenyl, Phenyl, Benzyl, R⁸ oder R⁹ ist, wobei C₁-C₆-Alkyl, C₃-C₇-Cycloalkyl, Phenyl und Benzyl gegebenenfalls mit Halogen, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, -NR⁵SO₂NR⁵R⁵, R⁸ oder R⁹ substituiert sind; R³ H, C₁-C₆-Alkyl, C₃-C₇-Cycloalkyl, Phenyl, Benzyl, Halogen, -CN, -OR⁷, -CO₂R⁵, -CONR⁵R⁵, R⁸ oder R⁹ ist, wobei C₁-C₆-Alkyl, C₃-C₇-Cycloalkyl, Phenyl und Benzyl gegebenenfalls mit Halogen, -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ oder R⁹ substituiert sind;
R⁴ Phenyl, Naphthyl oder Pyridyl ist, wobei jedes gegebenenfalls mit R⁸, Halogen, -CN, C₁-C₆-Alkyl, C₁-C₆-Halogenalkyl, C₃-C₇-Cycloalkyl, C₁-C₆-Alkoxy, -CONR⁵R⁵, OR¹¹, SoₓR⁶, O-(C₁-C₆-Alkylen) -CONR⁵R⁵, O- (C₁-C₆-Alkylen) -NR⁵R⁵ oder O- (C₁-C₆-Alkylen)-OR⁶ substituiert ist;
jedes R⁵ unabhängig entweder H, C₁-C₆-Alkyl oder C₃-C₇-Cycloalkyl ist oder, wenn zwei R⁵-Gruppen an das gleiche Stickstoffatom angeheftet sind, diese zwei Gruppen zusammengenommen mit dem Stickstoffatom, an das sie angeheftet sind, Azetidinyl, Pyrrolidinyl, Piperidinyl, Homopiperidinyl, Piperazinyl, Homopiperazinyl oder Morpholinyl darstellen, wobei Azetidinyl, Pyrrolidinyl, Piperidinyl, Homopiperidinyl, Piperazinyl, Homopiperazinyl und Morpholinyl gegebenenfalls mit C₁-C₆-Alkyl oder C₃-C₇-Cycloalkyl substituiert ist;
jedes R⁶ unabhängig entweder H, C₁-C₆-Alkyl oder C₃-C₇-Cycloalkyl ist;
R⁷ C₁-C₆-Alkyl oder C₃-C₇-Cycloalkyl ist;
R⁸ eine fünf- oder sechsgliedrige aromatische heterocyclische Gruppe ist, enthaltend (i) von 1 bis 4 Stickstoffheteroatome oder (ii) 1 oder 2 Stickstoffheteroatome und ein Sauerstoff- oder ein Schwefelheteroatom oder (iii) 1 oder 2 Sauerstoff- oder Schwefelheteroatome, wobei die heterocyclische Gruppe gegebenenfalls mit Halogen, Oxo, -CN, -COR⁵, -CONR⁵R⁵, -SO₂NR⁵R⁵, -NR⁵SO₂R⁵, -OR⁵, -NR⁵R⁵, -(C₁-C₆-Alkylen)-NR⁵R⁵, C₁-C₆-Alkyl, Fluor (C₁-C₆)alkyl oder C₃-C₇-Cycloalkyl substituiert ist;
R⁹ eine vier- bis siebengliedrige, gesättigte oder teilweise ungesättigte heterocyclische Gruppe ist, enthaltend (i) 1 oder 2 Stickstoffheteroatome oder (ii) 1 Stickstoffheteroatom und 1 Sauerstoff- oder 1 Schwefelheteroatom oder (iii) 1 Sauerstoff- oder Schwefelheteroatom, wobei die heterocyclische Gruppe gegebenenfalls mit Oxo, C₁-C₆-Alkyl, C₃-C₇-Cycloalkyl, -SO₂R⁵, -CONR⁵R⁵, -COOR⁵, -CO-(C₁-C₆-Alkylen) -OR⁵ oder -COR⁵ substituiert ist und gegebenenfalls an einem Kohlenstoffatom, das nicht einem Heteroatom benachbart ist, mit Halogen, -OR⁵, -NR⁵R⁵, -NR⁵COR⁵, -NR⁵COOR⁵, -NR⁵CONR⁵R⁵, -NR⁵SO₂R⁵ oder -CN substituiert ist; R¹⁰ C₁-C₆-Alkyl, substituiert mit R⁸, R⁹, -OR⁵, -CONR⁵R⁵, -NR⁵COR⁵ oder -NR⁵R⁵, ist;
R¹¹ Phenyl, gegebenenfalls substituiert mit Halogen, -CN, -COR⁵, -CONR⁵R⁵, -SO₂NR⁵R⁵, -NR⁵SO₂R⁵, -OR⁵, -NR⁵R⁵, - (C₁-C₆-Alkylen) -NR⁵R⁵, C₁-C₆-Alkyl, Halogen (C₁-C₆)alkyl oder C₃-C₇-Cylcoalkyl, ist; und
x und n unabhängig 0, 1 oder 2 sind.

2. Pharmazeutische Zusammensetzung, umfassend eine Verbindung der Formel (I) oder ein pharmazeutisch verträgliches Salz oder Solvat davon zusammen mit einem oder mehreren pharmazeutisch verträglichen Exzipiens/Exzipientien, Verdünnungsmittel(n) oder Träger(n).

3. Pharmazeutische Zusammensetzung nach Anspruch 2, umfassend ein oder mehrere zusätzliche therapeutische Mittel.

4. Verbindung der Formel (I) oder ein pharmazeutisch verträgliches Salz oder Solvat davon oder eine pharmazeutische Zusammensetzung nach Anspruch 2 oder 3 zur Verwendung als Medikament.

5. Verbindung der Formel (I) oder ein pharmazeutisch verträgliches Salz oder Solvat davon oder eine pharmazeutische Zusammensetzung nach Anspruch 2 oder 3 zur Verwendung als Reverse-Transkriptase-Inhibitor oder -Modulator.

6. Verbindung der Formel (I) oder ein pharmazeutisch verträgliches Salz oder Solvat davon oder eine pharmazeutische Zusammensetzung nach Anspruch 2 oder 3 zur Verwendung bei der Behandlung einer HIV- oder genetisch verwandten retroviralen Infektion oder eines resultierenden erworbenen Immundefektsyndroms (AIDS).

7. Verwendung einer Verbindung der Formel (I) oder eines pharmazeutisch verträglichen Salzes oder Solvats davon oder einer pharmazeutischen Zusammensetzung nach Anspruch 2 oder 3 zur Herstellung eines Medikaments mit Reverse-Transkriptaseinhibierender oder -modulierender Aktivität.

8. Verwendung einer Verbindung der Formel (I) oder eines pharmazeutisch verträglichen Salzes oder Solvats davon oder einer pharmazeutischen Zusammensetzung nach Anspruch 2 oder 3 zur Herstellung eines Medikaments zur Behandlung einer HIV- oder genetisch verwandten retroviralen Infektion oder eines resultierenden erworbenen Immundefektsyndroms (AIDS).

9. Verfahren zur Herstellung der Verbindung der Formel (I) oder eines pharmazeutisch verträglichen Salzes oder Solvats davon, welches umfasst:
(A) Umsetzung einer Verbindung der Formel (II) 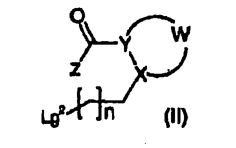 mit einem Amin der Formel (IV) 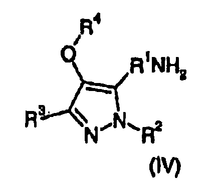 unter herkömmlichen Bedingungen;
(B) Umsetzung einer Verbindung der Formel (III) 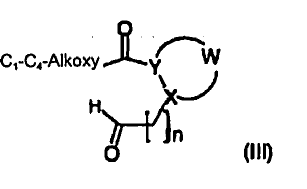 mit einem Amin der Formel (IV) 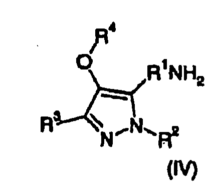 unter herkömmlichen Bedingungen;
(C) zur Herstellung einer Verbindung der Formel (I), in der R³ Halogen ist, Halogenieren einer Verbindung der Formel (XI) 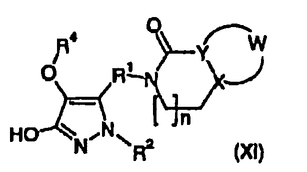 unter herkömmlichen Bedingungen;
(D) Interkonversion einer Verbindung der Formel (I) in eine andere Verbindung der Formel (I) oder
(E) Entschützen eines geschützten Derivats der Verbindung der Formel (I) und
gegebenenfalls Umwandeln einer Verbindung der Formel (I), hergestellt durch eines der Verfahren (A) bis (E), in ein pharmazeutisch verträgliches Salz oder Solvat davon.

## Revendications

1. Composé de formule (I) 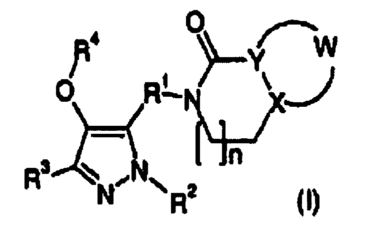 ou un de ses sels ou produits de solvatation pharmaceutiquement acceptables, formule dans laquelle :
W-X-Y définit un noyau phényle ou pyridyle, ledit noyau étant facultativement substitué avec un substituant CN ;
R¹ représente un groupe alkylène en C₁ à C₆ ;
R² représente H, un groupe alkyle en C₁ à C₆, alcényle en C₃ à C₆, alcynyle en C₃ à C₆, cycloalkyle en C₃ à C₇, cycloalcényle en C₃ à C₇, phényle, benzyle, R⁸ ou R⁹, lesdits groupes alkyle en C₁ à C₆, cycloalkyle en C₃ à C₇, phényle et benzyle étant facultativement substitués avec des substituants halogéno, -OR⁵, OR¹⁰, -CN, -CO₂R⁷, -OCONR⁵R⁶, -CONR⁵R⁶, -C (=NR⁵)NR⁶OR⁶, -CONR⁵NR⁵R⁵, -NR⁶R⁶, -NR⁵R¹⁰, -NR⁵COR⁵, -NR⁵COR⁶, -NR⁵COR¹⁰, -NR⁵CO₂R⁵, -NR⁵CONR⁵R⁵, -SO₂NR⁵R⁵, -NR⁶SO₂R⁶, -NR⁵SO₂NR⁶R⁵, R⁸ ou R⁹;
R³ représente H, un groupe alkyle en C₁ à C₆, cycloalkyle en C₃ à C₇, phényle, benzyle, halogéno, -CN, -OR⁷, -CO₂R⁶, -CONR⁵R⁵, R⁸ ou R⁹, lesdits groupes alkyle en C₁ à C₆, cycloalkyle en C₃ à C₇, phényle et benzyle étant facultativement substitués avec des substituants halogéno, -CN, -OR⁵, -CO₂R⁵, -CONR⁵R⁵, -OCONR⁵R⁵, -NR⁵CO₂R⁶, -NR⁶R⁶, -NR⁶COR⁵ , -SO₂NR⁵R⁵, NR⁶CONR⁵R⁵, -NR⁶SO₂R⁵, R⁸ ou R⁹ ;
R⁴ représente un groupe phényle, naphtyle ou pyridyle, chacun étant facultativement substitué avec des substituants R⁸, halogéno, -CN, alkyle en C₁ à C₆, halogénalkyle en C₁ à C₆, cycloalkyle en C₃ à C₇, alkoxy en C₁ à C₆, -CONR⁵R⁵, OR¹¹, SOₓR⁶, O (alkylène en C₁ à C₆)-CONR⁵R⁵, O (alkylène en C₁ à C₆)-NR⁵R⁵ ou O (alkylène en C₁ à C₆)-OR⁶ ;
chaque groupe R⁵ représente indépendamment H, un groupe alkyle en C₁ à C₆ ou cycloalkyle en C₃ ou C₇ ou bien, lorsque deux groupes R⁵ sont fixés au même atome d'azote, ces deux groupes, pris conjointement avec l'atome d'azote auquel ils sont fixés, représentent des groupes azétidinyle, pyrrolidinyle, pipéridinyle, homopipéridinyle, pipérazinyle, homopipérazinyle ou morpholinyle, lesdits groupes azétidinyle, pyrrolidinyle, pipéridinyle, homopipéridinyle, pipérazinyle, homopipérazinyle et morpholinyle étant facultativement substitués avec des substituants alkyle en C₁ à C₆ ou cycloalkyle en C₃ à C₇ ;
chaque groupe R⁶ représente indépendamment H, un groupe alkyle en C₁ à C₆ ou cycloalkyle en C₃ à C₇ ;
R⁷ représente un groupe alkyle en C₁ à C₆ ou cycloalkyle en C₃ à C₇ ;
R⁸ représente un groupe hétérocyclique aromatique penta- ou hexagonal contenant (i) 1 à 4 hétéroatomes d'azote ou (ii) 1 ou 2 hétéroatomes d'azote et 1 hétéroatome d'oxygène ou un hétéroatome de soufre ou (iii) 1 ou 2 hétéroatomes d'oxygène ou de soufre, ledit groupe hétérocyclique étant facultativement substitué avec des substituants halogéno, oxo, -CN, -COR⁵, -CONR⁵R⁵, -SO₂NR⁵R⁵, -NR⁵SO₂R⁵, -OR⁵, -NR⁵R⁵, -(alkylène en C₁ à C₆) -NR⁵R⁵, alkyle en C₁ à C₆, fluoralkyle en C₁ à C₆ ou cycloalkyle en C₃ à C₇ ;
R⁹ représente un groupe hétérocyclique tétra- à heptagonal, saturé ou partiellement insaturé, contenant (i) 1 ou 2 hétéroatomes d'azote ou (ii) 1 hétéroatome d'azote et 1 hétéroatome d'oxygène ou 1 hétéroatome de soufre ou (iii) 1 hétéroatome d'oxygène ou de soufre, ledit groupe hétérocyclique étant facultativement substitué avec des substituants oxo, alkyle en C₁ à C₆, cycloalkyle en C₃ à C₇, -SO₂R⁵, -CONR⁵R⁵, - COOR⁵, -CO-(alkylène en C₁ à C₆) -OR⁵ ou -COR⁵ et étant facultativement substitué sur un atome de carbone qui n'est pas adjacent à un hétéroatome avec un substituant halogéno, -OR⁵, -NR⁵R⁵, -NR⁵COR⁵, -NR⁵COOR⁵, -NR⁵CONR⁵R⁵, -NR⁵SO₂R⁵ ou -CN ;
R¹⁰ représente un groupe alkyle en C₁ à C₆ substitué avec des substituants R⁶, R⁹, -OR⁵, -CONR⁵R⁵, -NR⁵COR⁵ ou -NR⁵R⁵;
R¹¹ représente un groupe phényle facultativement substitué avec des substituants halogéno, -CN, -COR⁵, -CONR⁵R⁵, -SO₂NR⁵R⁵, -NR⁵SO₂R⁵, -OR⁶, -NR⁶R⁵, - (alkylène en C₁ à C₆) -NR⁵R⁵, alkyle en C₁ à C₆, halogénalkyle en C₁ à C₆ ou cycloalkyle en C₃ à C₇ ; et
x et n sont égaux indépendamment à 0, 1 ou 2.

2. Composition pharmaceutique comprenant un composé de formule (I) ou un de ses sels ou produits de solvatation pharmaceutiquement acceptables, conjointement avec un ou plusieurs excipients, diluants, ou supports pharmaceutiquement acceptables.

3. Composition pharmaceutique suivant la revendication 2, comprenant un ou plusieurs agents thérapeutiques supplémentaires.

4. Composé de formule (I) ou un de ses sels ou produits de solvatation pharmaceutiquement acceptables, ou composition pharmaceutique suivant la revendication 2 ou 3, destiné à être utilisé comme médicament.

5. Composé de formule (I) ou un de ses sels ou produits de solvatation pharmaceutiquement acceptables, ou composition pharmaceutique suivant la revendication 2 ou 3, destiné à être utilisé comme inhibiteur ou modulateur de la transcriptase inverse.

6. Composé de formule (I) ou un de ses sels ou produits de solvatation pharmaceutiquement acceptables, ou composition pharmaceutique suivant la revendication 2 ou 3, destiné à être utilisé dans le traitement d'une infection par le VIH ou d'un rétrovirus génétiquement apparenté, ou d'un syndrome d'immunodéficience acquise (SIDA) résultant.

7. Utilisation d'un composé de formule (I) ou d'un de ses sels ou produits de solvatation pharmaceutiquement acceptables, ou d'une composition pharmaceutique suivant la revendication 2 ou 3, pour la production d'un médicament ayant une activité d'inhibition ou de modulation de la transcriptase inverse.

8. Utilisation d'un composé de formule (I) ou d'un de ses sels ou produits de solvatation pharmaceutiquement acceptables, ou d'une composition pharmaceutique suivant la revendication 2 ou 3, pour la production d'un médicament destiné au traitement d'une infection par le VIH ou d'un rétrovirus génétiquement apparenté, ou d'un syndrome d'immunodéficience acquise (SIDA) résultant.

9. Procédé pour la préparation du composé de formule (I) ou d'un de ses sels ou produits de solvatation pharmaceutiquement acceptables, qui comprend :
(A) la réaction d'un composé de formule (II) 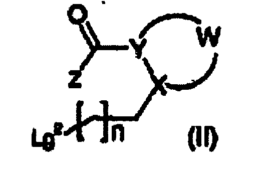 avec une amine de formule (IV) 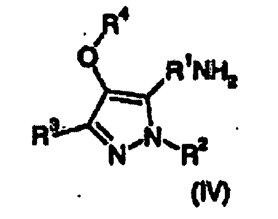 dans des conditions classiques ;
(B) la réaction d'un composé de formule (III) 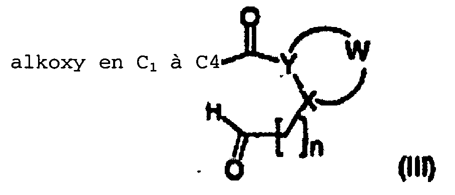 avec une amine de formule (IV) 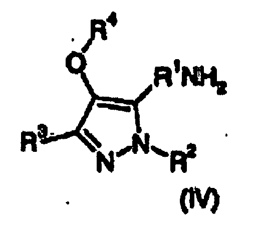 dans des conditions classiques ;
(C) pour la préparation d'un composé de formule (I) dans laquelle R³ représente un groupe halogéno, l'halogénation d'un composé de formule (XI) 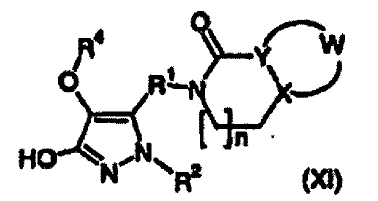 dans des conditions classiques ;
(D) l'interconversion d'un composé de formule (I) en un autre composé de formule (I) ; ou
(E) la suppression de protection d'un dérivé protégé d'un composé de formule (I) et
facultativement la conversion d'un composé de formule (I) préparé par l'un quelconque des procédés (A) à (E) en un de ses sels ou produits de solvatation pharmaceutiquement acceptables.
